# EUROPEAN PATENT APPLICATION

(11) **EP 2 540 327 A1**
(43) Date of publication of application: **02.01.2013**
(21) Application number: 11744704.5
(22) Date of filing: 17.02.2011
(51) Int. Cl.: A61M 1/18, A61L 31/00, A61M 1/14

(54) **MEDICAL DEVICE AND HOLLOW FIBER MEMBRANE MEDICAL DEVICE**

(30) Priority: 30.03.2010 JP 2010079471; 22.02.2010 JP 2010036339
(71) Applicant: Asahi Kasei Kuraray Medical Co., Ltd., Chiyoda-ku Tokyo 101-8101 (JP)
(72) Inventor: KAWATANI, Katsuji, Tokyo 101-8101 (JP); MIYAZAKI, Shinji, Tokyo 101-8101 (JP); KOIZUMI, Toshinori, Tokyo 101-8101 (JP)
(74) Representative: von Kreisler Selting Werner
(86) International application number: PCT/JP2011/053383
(87) International publication number: WO 2011/102418

(57) **Abstract**

It is intended to provide a highly reliable medical device, for example, hollow fiber membrane-type medical device, which can effectively prevent the occurrence of dielectric breakdown in an electron beam irradiation sterilization step. The present invention provides a hollow fiber membrane-type medical device comprising: a container having an inner space molded with an insulating material; a filter medium loaded in the inner space; an inlet provided on a container wall surface defining the inner space and intended for fluid influx into the container; an outlet provided on the container wall surface defining the inner space and intended for fluid efflux from the container, wherein the inlet and/or the outlet have a portion consisting of a polymer material having volume resistivity of 5 × 10¹³ Ω·cm or smaller, and the medical device has been irradiated with an electron beam.

## Description

### Technical Field

The present invention relates to a medical device and a hollow fiber membrane-based medical device.

### Background Art

In recent years, there has been an advance in the medical application of techniques of, particularly, hollow fiber membranes, among medical devices comprising a predetermined filter medium in the inner space. Such hollow fiber membrane-based medical devices are frequently used in, for example, artificial kidneys for use in the hemodialysis therapy of chronic renal failure patients, plasma separators for use in plasma exchange therapy involving discarding a patient's plasma and infusing instead fresh frozen plasma or an albumin solution, and plasma fractionators for use in double filtration plasma exchange therapy involving removing high-molecular-weight substances in the patient's plasma.

The hollow fiber membrane-based medical devices come into contact with the patient's body fluid, such as blood, and thus, must be sterilized for use.
Examples of sterilization methods may include heat sterilization, chemical sterilization, and irradiation sterilization. These sterilization methods are appropriately selected in consideration of the thermal and chemical properties of the hollow fiber membrane-based medical devices.
Particularly, electron beam sterilization, one approach of the irradiation sterilization, is known to be highly useful, because this sterilization method is free from exposure to high temperature as in heat sterilization, chemical agents remaining in medical devices as in chemical sterilization, or the need for storing radioactive substances in large amounts as in γ ray sterilization, another approach of the irradiation sterilization (see e.g., Patent Document 1).

Electron beams, however, are less permeable to objects than γ rays, and the distance penetrated thereby depends on the density of a substance to be irradiated. For this reason, conventional targets for electron beam sterilization are mainly those having a relatively uniform shape and composed of a single member, such as surgical gloves or surgical gowns. When the hollow fiber membrane-based medical device, which is thick and has a high-density portion, is subjected to electron beam irradiation, this irradiation might insufficiently permeate some portions and cause a large dose distribution (ratio between the maximum dose and the minimum dose) among sites, resulting in the manifestation of problems such as material deterioration or eluted materials.
Specifically, irradiation on the basis of the maximum dose causes insufficient sterilization at a position of the minimum dose. On the contrary, reliable sterilization by irradiation on the basis of a site of the minimum dose may cause excessive irradiation at a position of the maximum dose, resulting in material deterioration or staining.
Particularly, if a hydrophilic polymer used as a constituent material in the hollow fiber membrane becomes deteriorated, its hydrophilicity is impaired. The resulting device has reduced blood compatibility.
As described above, it is not easy to uniformly irradiate objects having a complicated shape and different densities among sites, as in the hollow fiber membrane-based medical devices, with an electron beam. Thus, the electron beam sterilization is accompanied by problems based on nonuniform irradiation.

Thus, as measures for uniformly irradiating objects having a complicated shape and different densities among sites, as in the hollow fiber membrane-based medical devices, with an electron beam, studies have been made mainly from two viewpoints: a materials chemical approach and an approach based on a sterilization step.
First, for the materials chemical approach, a large number of techniques involving kneading an additive such as a radical-trapping agent or an antioxidant into a resin material or allowing such an additive to coexist in the vicinity of resin are known and have been studied extensively as methods for suppressing deterioration during exposure to radiation including electron beams. These materials chemical approaches have such advantages that they eliminate the need for drastically renovating irradiation facilities and achieve efficient production without prolonging irradiation cycle time.
Meanwhile, the problems of the materials chemical approach are that: many additives cannot be adopted for extracorporeal circulation-based blood purification apparatuses from a safety standpoint; and approaches for the hollow fiber membrane-based medical devices have mostly been made concrete as improvements dedicated to material deterioration during γ ray sterilization and are thus utilized in a limited range (see e.g., Patent Documents 2 and 3).

Next, a method involving raising an accelerating voltage has received attention as the approach based on a sterilization step.
For example, there are disclosed a technique of performing irradiation at high accelerating voltage for the electron beam sterilization of hollow fiber membrane-based dialysis apparatuses or artificial lungs, while reducing dose distribution using a shielding material (see e.g., Patent Document 4) and an irradiation method combining an entire irradiation step and a partial shielding step (see e.g., Patent Document 5).
Also, Patent Document 1 discloses a technique of achieving a uniform irradiation dose as a whole by irradiating a hollow fiber membrane-based module having the particular product of density and thickness with an electron beam in 3 or more directions.
These approaches based on a sterilization step can overcome the challenge of reducing dose distribution. However, all of them disadvantageously have poor workability and poor efficiency in the practice of sterilization methods for mass products.

Furthermore, studies have been made in terms of both the materials chemical approach and the approach based on a sterilization step to prevent material deterioration and achieve a uniform absorbed dose of electron beams.
However, the effect of improving (reducing) the adsorbed dose distribution of electron beams becomes insufficient with the firm commitment to material protection from a materials chemical standpoint. On the contrary, the effect of efficiently treating objects to be irradiated becomes insufficient with the firm commitment to methods for individually irradiating objects to be irradiated or facilities therefor from the viewpoint of a uniform absorbed dose.

Aside from the materials chemical approach or the approach based on a sterilization step, focusing on the package form of the hollow fiber membrane-based medical device, an approach involves housing a stack structure in an electron beam-permeable case, followed by irradiation sterilization with an electron beam, the stack structure comprising: a gap layer with a particular density containing no hollow fiber membrane-based medical device; and body fluid treatment device layers with a particular density comprising tubular hollow fiber membrane-based medical devices arranged in parallel. There is disclosed a technique of thereby protecting the materials for the hollow fiber membrane-based medical devices themselves, while achieving a uniform absorbed dose (see e.g., Patent Literature 6).

As described above, the problems associated with a nonuniform absorbed dose in the electron beam irradiation of objects to be irradiated having a complicated shape and largely different densities among sites, as in the hollow fiber membrane-based medical devices, and with material deterioration have previously been tackled by means of a devised package form, increased accelerating voltage, or multidirectional irradiation.

Meanwhile, aside from these problems associated with material deterioration or a nonuniform absorbed dose in the electron beam sterilization step as described above, a new challenge to the electron beam sterilization step has arisen in recent years. Specifically, an insulating polymer material such as polyolefin resin is generally used as a constituent material in the hollow fiber membrane-based medical devices. Upon electron beam irradiation, electric charge accumulates in such an insulating polymer material. The accumulation of electric charge above a certain level causes undesired dielectric breakdown (see e.g., Patent Documents 7 and 8).

### Prior Art Documents

### Patent Document

Patent Document 1: Japanese Patent Laid-Open No. 2000-325434
Patent Document 2: Japanese Patent No. 3076080
Patent Document 3: Japanese Patent No. 3432240
Patent Document 4: Japanese Patent Laid-Open No. 8-275991
Patent Document 5: Japanese Patent Laid-Open No. 2000-334028
Patent Document 6: International Publication No. 2009/017227
Patent Document 7: Japanese Patent Laid-Open No. 2000-001561
Patent Document 8: Japanese Patent Laid-Open No. 2001-324656

### Summary of Invention

### Problems to be Solved by the Invention

Only in a few cases, medical devices having a complicated shape such as medical devices having a predetermined filter medium in the inner space, particularly, hollow fiber membrane-based medical devices, are actually irradiated with an electron beam. Issues such as the accumulation of electric charge in objects to be irradiated and dissipation thereof have not yet been studied sufficiently.

Nonuniform irradiation during the electron beam irradiation of medical devices having a complicated shape and largely different densities among sites has previously been tackled by means of a devised package form, increased accelerating voltage, or multidirectional irradiation as strategies for solving this problem, as described above. Some of these strategies might promote the accumulation of electric charge in a portion or the whole of an object to be irradiated containing an insulating constituent member.

The occurrence of dielectric breakdown in a portion of a medical device, as described above, might not only cancel the sterility of the medical device but also significantly jeopardize persons receiving treatment with the medical device. For electron beam irradiation sterilization, it is thus required to overcome the challenge of achieving a uniform absorbed dose as well as to study the issues associated with the accumulation and dissipation of electric charge.

The present inventors have conducted diligent studies on the electron beam irradiation sterilization of medical devices.
For example, when a hollow fiber membrane-based medical device is sterilized by electron beam irradiation, its irradiation dose is determined in consideration of the deterioration of members and sterility and is generally approximately 15 to 20 kGy for dry or semidry type and approximately 25 kGy even for wet type.

In order to study the problems associated with the accumulation of electric charge, the present inventors have prepared, on trial, a semidry-type hollow fiber membrane-based medical device irradiated with 35 kGy electron beams, which are an alleged excessive dose in conventional techniques, and have visually observed it. As a result, the present inventors have found dielectric breakdown signs in a portion of the hollow fiber membrane-based medical device thus sterilized.
Specifically, each dialyzer was prepared using a bundle of the hollow fiber membrane containing an aqueous glycerin solution in the hollow fiber membrane obtained by a dry/wet spinning method using a dope containing polysulfone and polyvinylpyrrolidone. Nozzles and ports were separately capped with a plug made of polyethylene. The dialyzer was packaged in a sterilization bag having a polyethylene/nylon bilayer structure and then irradiated with 35 kGy electron beams.
After the irradiation, each dialyzer was examined for the presence or absence of abnormalities resulting from electron beam irradiation itself or secondarily resulting from temporary electric charge accumulation. As a result, in some dialyzers, dielectric breakdown signs were found in the central portion of the top of the plug attached to the port on the side of the tubular container.

Thus, in light of these problems of the conventional techniques, an object of the present invention is to provide a medical device, particularly, a hollow fiber membrane-based medical device, which can effectively prevent the occurrence of dielectric breakdown even when sterilized by electron beam irradiation at a high dose.

### Means for Solving the Problems

As a result of conducting diligent studies to attain the object, the present inventors have found that the occurrence of dielectric breakdown can be prevented by specifying at least the partial volume resistivity of components, particularly, plugs, constituting a medical device, particularly, a hollow fiber membrane-based medical device. Based on these findings, the present invention has been completed.
Specifically, the present invention is as follows:

[1] A hollow fiber membrane-based medical device comprising:
   a tubular container;
   a bundle of a hollow fiber membrane loaded along a longitudinal direction of the tubular container and having both ends fixed to both ends of the tubular container, respectively;
   a potting resin embedding both ends of the bundle of the hollow fiber membrane, respectively, so that the ends are fixed to the both ends of the tubular container, respectively;
   a header facing both end faces of the hollow fiber membrane and provided at the both ends of the tubular container, respectively, the header having a nozzle serving as a fluid inlet and/or outlet;
   a port provided on a side of the tubular container and serving as a fluid inlet and/or outlet;
   a nozzle plug removably attached to the respective nozzles; and
   a port plug removably attached to the port,
      wherein the port plug has a portion formed of a polymer material having a volume resistivity of 5 × 10¹³ Ω·cm or smaller, and
   the hollow fiber membrane-based medical device is irradiated with an electron beam.
[2] The hollow fiber membrane-based medical device according to above [1], wherein the port plug is formed of a polymer material having a volume resistivity of 5 × 10¹³ Ω·cm or smaller.
[3] The hollow fiber membrane-based medical device according to above [1] or [2], wherein the nozzle plug have a portion formed of a polymer material having a volume resistivity of 5 × 10¹³ Ω·cm or smaller.
[4] The hollow fiber membrane-based medical device according to any one of above [1] to [3], wherein the nozzle plug is formed of a polymer material having a volume resistivity of 5 × 10¹³ Ω·cm or smaller.
[5] The hollow fiber membrane-based medical device according to any one of above [1] to [4], wherein the port plug has, at least in a partial top portion thereof, the portion formed of a polymer material having a volume resistivity of 5 × 10¹³ Ω·cm or smaller.
[6] The hollow fiber membrane-based medical device according to any one of above [1] to [5], further comprising
   a first space between an internal side of the hollow fiber membrane and an inner surface of the header, and
   a second space between an external side of the hollow fiber membrane and an inner surface of the tubular container,
   wherein the port plug has, at least in a partial top portion thereof, the portion formed of a polymer material having a volume resistivity of 5 × 10¹³ Ω·cm or smaller, wherein the portion is brought into contact on an outer surface side of the port plug with an external space of the hollow fiber membrane-based medical device and on an inner surface side of the port plug with the second space in a seamless way.
[7] The hollow fiber membrane-based medical device according to any one of above [1] to [6], wherein the port plug has, only in a top portion thereof, the portion formed of a polymer material having a volume resistivity of 5 × 10¹³ Ω·cm or smaller.
[8] A medical device comprising:
   a container having an inner space molded with an insulating material;
   a filter medium loaded in the inner space;
   an inlet provided on a container wall surface which defines the inner space and flowing a fluid into the container;
   an outlet provided on a container wall surface which defines the inner space and effusing a fluid from the container,
   wherein the inlet and/or the outlet have a portion formed of a polymer material having a volume resistivity of 5 × 10¹³ Ω·cm or smaller, and
   the medical device is irradiated with an electron beam.

### Advantageous Effects of Invention

The present invention can provide a highly reliable medical device, particularly, hollow fiber membrane-based medical device, which can effectively prevent the occurrence of dielectric breakdown in an electron beam irradiation sterilization step.

### Brief Description of Drawings

[Figure 1] Figure 1 illustrates a partially sectional diagram schematically showing one example of a hollow fiber membrane-based medical device of the present embodiment.
[Figure 2] Figure 2 illustrates a schematic sectional diagram of a nozzle constituting the hollow fiber membrane-based medical device of the present embodiment.
[Figure 3] Figure 3 illustrates a schematic sectional diagram of one example of a nozzle plug.
[Figure 4] Figure 4 illustrates a schematic diagram showing the constitution of a port constituting the hollow fiber membrane-based medical device of the present embodiment.
[Figure 5] Figure 5(A) illustrates a schematic sectional diagram of one example of a port plug. Figure 5(B) illustrates a schematic sectional diagram of the port plug in a state attached to the port.
[Figure 6] Figure 6(A) illustrates a schematic sectional diagram of another example of a port plug. Figure 6(B) illustrates a schematic sectional diagram of the port plug of another example in a state attached to the port.
[Figure 7] Figures 7(A) to 7(T) illustrate schematic sectional diagrams of specific examples of port plugs.

### Mode for Carrying Out the Invention

Hereinafter, the mode for carrying out the present invention (hereinafter, referred to as the "present embodiment") will be described with reference to the drawings.
It is to be understood that the present invention is not intended to be limited to the descriptions below, and various changes may be made therein without departing from the spirit of the present invention.
In the drawings, the same reference numerals will be used to designate the same components, so that overlapping descriptions will be omitted. Positional relationships indicated by the terms "upper", "lower", "right", "left", and the like are based on those shown in the drawings, unless otherwise specified. In addition, the dimensional ratios of the drawings are not intended to be limited to those shown in the drawings.

### [Medical device]

The medical device of the present embodiment comprises: a container having an inner space molded with an insulating material; a filter medium loaded in the inner space; an inlet provided on a container wall surface which defines the inner space and flowing a fluid into the container; and an outlet provided on a container wall surface which defines the inner space and effusing a fluid from the container. The inlet and/or the outlet each have a predetermined plug as a portion formed of a polymer material having a volume resistivity of 5 × 10¹³ Ω·cm or smaller. In addition, the medical device of the present embodiment is sterilized by electron beam irradiation.

Examples of typical embodiments of the medical device of the present embodiment may include a hollow fiber membrane-based medical device comprising: a tubular container; a bundle of a hollow fiber membrane loaded along a longitudinal direction of the tubular container and having both ends fixed to both ends of the tubular container, respectively; a potting resin embedding both ends of the bundle of the hollow fiber membrane, respectively, so that the ends are fixed to both ends of the tubular container, respectively; a header facing both end faces of the hollow fiber membrane and provided at both ends of the tubular container, respectively, the header having a nozzle serving as a fluid inlet and/or outlet; a port provided on a side of the tubular container and serving as a fluid inlet and/or outlet; a nozzle plug removably attached to the respective nozzles; and a port plug removably attached to the port, wherein the port plug has a portion formed of a polymer material having a volume resistivity of 5 × 10¹³ Ω·cm or smaller, and the hollow fiber membrane-based medical device is sterilized by electron beam irradiation.
In this constitution, the container having the inner space molded with the insulating material corresponds to the tubular container, and the filter medium corresponds to the bundle of the hollow fiber membrane.
Also, the inlet and/or the outlet correspond to a port with a port plug, preferably, a nozzle with nozzle plug in addition to the port with the port plug. A portion or the whole of these plugs corresponds to the " portion formed of a polymer material having a volume resistivity of 5 × 10¹³ Ω·cm or smaller".

In the description below, a preferable example of the medical device of the present embodiment, i.e., a hollow fiber membrane-based medical device comprising a bundle of the hollow fiber membrane loaded along the longitudinal direction in the tubular container will be described with reference to the drawings. However, the medical device of the present embodiment is not intended to be limited to the example below and needs only to be a medical device comprising a predetermined filter medium loaded in the container having the inner space molded with the insulating material and the predetermined inlet and outlet on the container wall surface.

### [Hollow-fiber membrane-based medical device]

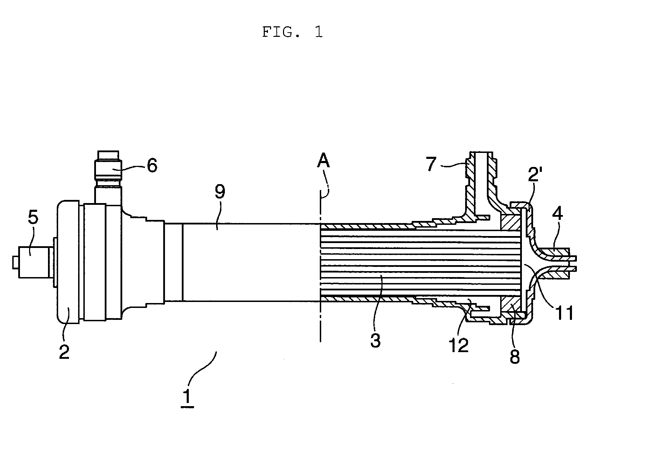

Figure 1 illustrates a partially sectional diagram schematically showing one example of a hollow fiber membrane-based medical device of the present embodiment.
Figure 1 shows a schematic sectional view of the hollow fiber membrane-based medical device on the right side of the alternate long and short dash line A shown at the center and shows a schematic perspective view of the hollow fiber membrane-based medical device on the left side of the alternate long and short dash line A.
In the hollow fiber membrane-based medical device 1, bundle 3 of a hollow fiber membrane is loaded along the longitudinal direction of tubular container 9.
Both ends of the bundle of the hollow fiber membrane 3 are embedded in potting resins 8, respectively, so that the internal side of the hollow fiber membrane 3 is isolated from the external side thereof and that the hollow fiber membrane 3 is fixed at both ends of the tubular container 9.
Headers 2 and 2' having a nozzle 4 or 5 serving as a fluid inlet and/or outlet are provided on the end faces of the potting resins 8, respectively, where the internal side of the hollow fiber membrane 3 is open.
At least one of ports 6 and 7 serving as a fluid inlet and/or outlet is provided on the side of the tubular container 9.
Nozzle plugs and port plugs described later can be attached to the nozzles 4 and 5 and the ports 6 and 7, respectively. A first space 11 is provided between the internal side of the hollow fiber membrane and the inner surface of each header, while a second space 12 is provided between the external side of the hollow fiber membrane 3 and the inner surface of the tubular container.

### (Container having inner space molded with insulating material: Tubular container)

The tubular container 9, which is a container having the inner space molded with the insulating material, is used for accommodating the hollow fiber membrane described later in the hollow fiber membrane-based medical device of the present embodiment. The tubular container 9 is not particularly limited by shape, dimension, etc., and any of those conventionally known in the art can be applied to the present invention.
The insulating material refers to a material having a volume resistivity larger than 5 × 10¹³ Ω·cm.
Examples of the insulating material for the tubular container 9 may include polypropylene resins, polystyrene resins, polymethyl methacrylate resins, polyethylene terephthalate resins, nylon 6 resins, polysulfone resins, polyacrylonitrile resins, polycarbonate resins, ABS resins, and styrene-butadiene copolymer resins.
Particularly, the polypropylene resins, the polystyrene resins, the polyacrylonitrile resins, and the styrene-butadiene copolymer resins are preferable because these resins require only low cost, are highly versatile in the field of medical members, and have proven high safety. The styrene-butadiene copolymer resins are particularly preferable, though the insulating material according to the present invention is not limited to them.

### (Filter medium: Hollow fiber membrane)

The hollow fiber membrane 3, which is an embodiment of the filter medium constituting the hollow fiber membrane-based medical device of the present embodiment, performs the component separation (filtration, adsorption, etc.) of a solution to be treated in the hollow fiber membrane-based medical device of the present embodiment.
The hollow fiber membrane 3 is not particularly limited by shape, dimension, fractionation characteristics, etc. and can be selected appropriately according to the use purpose.
A material for the hollow fiber membrane 3 is selected from materials sterilizable with an electron beam. Examples thereof may include, but not limited to, cellulose polymers, polysulfone polymers, polyacrylonitrile polymers, polymethyl methacrylate polymers, polyvinyl polymers (including ethylene vinyl alcohol copolymers), polyamide polymers, polyester polymers, and polyolefin polymers.
Particularly, polysulfone polymers, which are aromatic compounds, are preferable because these polymers are particularly excellent in radiation resistance, also very resistant to heat or chemical treatment, and also excellent in safety.

The hollow fiber membrane 3 can be produced by a technique conventionally known in the art. A resin material is dissolved in a predetermined solvent to prepare a dope, which can then be spun to prepare the hollow fiber membrane 3.
When a hydrophobic polymer is used as a base material for the hollow fiber membrane 3, a hydrophilic polymer is further used. In general, these polymers are blended using a common solvent therefor to prepare a dope, with which the hollow fiber membrane 3 is formed so that hydrophilicity is imparted thereto.
Examples of the hydrophilic polymer may include polyvinylpyrrolidone (PVP), polyethylene glycol, polyvinyl alcohol, and polypropylene glycol.
Particularly, polyvinylpyrrolidone is preferable in terms of hydrophilicity-imparting effect or safety, though the hydrophilic polymer according to the present invention is not particularly limited to them.
When the hydrophilic polymer is PVP, examples of the common solvent may include solvents such as dimethylacetamide (hereinafter, referred to as DMAC), dimethyl sulfoxide, N-methyl-2-pyrrolidone, dimethylformamide, sulfolane, and dioxane, and a mixed solvent of two or more of these solvents.
The dope may be supplemented with a predetermined additive such as water to control the pore size of the hollow fiber membrane 3 of interest.

The hollow fiber membrane 3 is formed using a tube-in-orifice-type spinneret. The dope and an inner solution for the dope are simultaneously discharged into air from an orifice of the spinneret and from a tube, respectively.
Water or an inner solution composed mainly of water can be used as the inner solution. Its composition or the like can be determined according to the permeability of the hollow fiber membrane of interest. In general, a mixed solution of water with the solvent used in the dope is preferably used. For example, an aqueous solution containing 0 to 65% by mass of DMAC is used.
The dope thus discharged from the spinneret together with the inner solution is traveled in an air gap portion and introduced and dipped in a coagulation bath (composed mainly of water) placed below the spinneret to complete coagulation.
After a washing step, etc., the hollow fiber membrane in a wet state is wound up using a winding machine to obtain a bundle of the hollow fiber membrane, which is then dried. Alternatively, after the washing step, the hollow fiber membrane may be dried in a dryer to obtain a bundle of the hollow fiber membrane.

Referring to Figure 1, the bundle of the hollow fiber membrane 3 is loaded along the longitudinal direction of the tubular container 9 and fixed at both ends of the tubular container 9.
For example, the bundle of the hollow fiber membrane 3 is inserted in the tubular container 9 having a fluid inlet and/or outlet. The potting resins 8 are injected to both ends of the bundle of the hollow fiber membrane 3. The resulting tubular container 9 is centrifuged around the central axis in the longitudinal direction so that layers of the potting resins 8 are formed at both ends of the bundle of the hollow fiber membrane 3 to fix these ends to both ends of the tubular container, respectively, by sealing.
After the fixation of the bundle of the hollow fiber membrane 3, redundant potting resins are removed by cutting so that the hollow portion of the hollow fiber membrane 3 is open at both ends. The headers 2 and 2' having a fluid inlet/outlet are attached thereto to thereby load the bundle of the hollow fiber membrane 3 in the tubular container 9. As a result, the first space 11 is formed between the internal side of the hollow fiber membrane 3 and the inner surface of each header, while the second space 12 is formed between the external side of the hollow fiber membrane 3 and the inner surface of the tubular container 9.

### (Potting resin)

The potting resins 8 have the function of sealing both ends of the bundle of the hollow fiber membrane 3.
Examples of materials for the potting resins 8 may include, but not limited to, polyurethane resins, epoxy resins, and silicon resins.

### (Header)

The headers 2 and 2' face both end faces of the hollow fiber membrane 3 and are provided at both ends of the tubular container 9, respectively.
Examples of materials for the headers 2 and 2' may include polypropylene resins, polystyrene resins, polymethyl methacrylate resins, polyethylene terephthalate resins, nylon 6 resins, polysulfone resins, polyacrylonitrile resins, polycarbonate resins, ABS resins, and styrene-butadiene copolymer resins.
Particularly, the polypropylene resins, the polystyrene resins, the polyacrylonitrile resins, and the styrene-butadiene copolymer resins are preferable because these resins require only low cost, are highly versatile in the field of medical members, and have proven high safety. The styrene-butadiene copolymer resins are particularly preferable, though the material according to the present invention is not limited to them.

### (Header nozzle)

The nozzles 4 and 5 of the headers 2 and 2' each constitute an inlet and/or an outlet for flowing a fluid into the tubular container and/or effusing a fluid from the container, together with nozzle plugs described later.

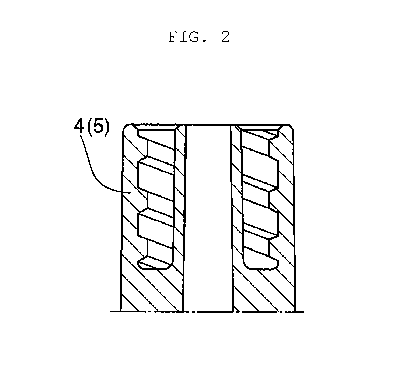

Figure 2 illustrates a schematic sectional diagram of the nozzles 4 and 5 of the headers 2 and 2'.
For example, structures described in 4.4.3 (connected parts on the blood side of hemodialyzers, hemodialysis filters, and hemofilters) of JIS T 3250:2005 (hemodialyzers, hemodialysis filters, hemofilters, and blood concentrators) can be applied to the structures of the nozzles 4 and 5.

### (Nozzle plug)

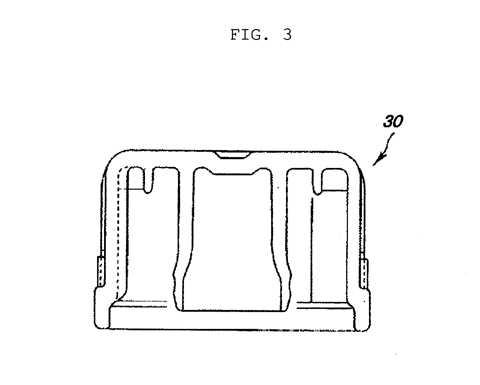

Figure 3 illustrates a schematic sectional diagram of each nozzle plug 30.
The nozzle plugs 30 have the function of blocking the openings of the nozzles 4 and 5 from the outside and may each have, on the internal side of its top portion, a cylindrical projection to be interlocked with the lateral surface of each of the nozzles 4 and 5 and a cylindrical projection to be interlocked with the hollow portion of each nozzle.
The nozzle plugs 30 can be sterilized, in a state attached to the nozzles 4 and 5, by electron beam irradiation described later to thereby maintain the sterilized state of the first space 11 shown in Figure 1.
In the hollow fiber membrane-based medical device of the present embodiment, the inlet and/or the outlet have a portion formed of a polymer material having a volume resistivity of 5 × 10¹³ Ω·cm or smaller. The portion having a volume resistivity of 5 × 10¹³ Ω·cm or smaller may be a portion or the whole of each nozzle plug 30.
Specific examples of materials for the nozzle plugs 30 may include, but not limited to, polyethylene resins, polypropylene resins, polycarbonate resins, polystyrene resins, polyamide resins, polyacetal resins, and silicon resins.
Particularly, the polyethylene resins and the polypropylene resins are preferable because these resins have adhesion properties or plugging properties based on moderate hardness and are already versatile as wrapping-type plugs (described later) or squeezed-type plugs (described later).

### (Port)

The ports 6 and 7 each constitute an inlet and/or an outlet for flowing the fluid into the tubular container and/or for effusing the fluid from the container, together with port plugs described later.

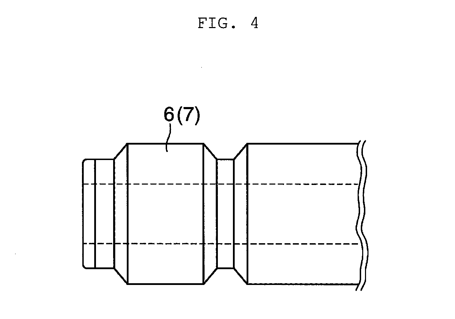

Figure 4 illustrates a schematic perspective diagram of the ports 6 and 7 provided on the side of the tubular container 9 constituting the hollow fiber membrane-based medical device of the present embodiment.
For example, structures described in 4.4.4 (connected parts on the dialysate side of hemodialyzers and hemodialysis filters) of JIS T 3250:2005 (hemodialyzers, hemodialysis filters, hemofilters, and blood concentrators) can be applied to the structures of the ports 6 and 7.
The ports 6 and 7, as shown in the broken lines in Figure 4, are in a tubular form having an internal hollow portion communicated with the inside of the tubular container 9 and can be opened and closed by the attachment and removal of their respective port plugs described later.

### (Port plug)

The port plugs open and close the ports 6 and 7 through the attachment and removal thereof.

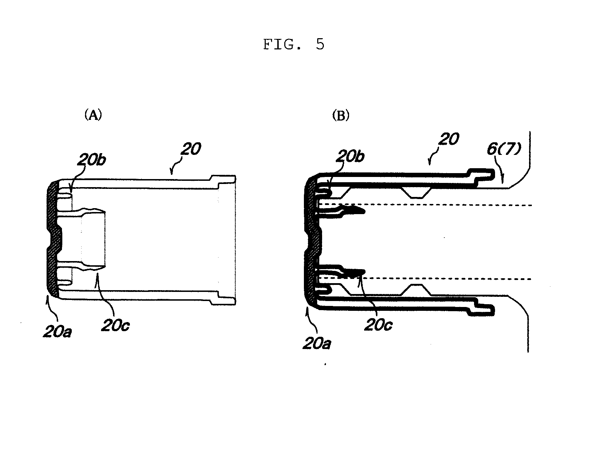

Figure 5(A) illustrates a schematic sectional diagram of each port plug 20. Figure 5(B) illustrates a schematic sectional diagram of the port plug 20 in a state attached to the port 6 or 7.
The port plugs 20, as shown in Figure 5(A), each have a top portion 20a having the function of blocking the opening of each of the ports 6 and 7 from the outside and may each have a cylindrical projection 20b to be interlocked with the lateral surface of each of the ports 6 and 7 and a cylindrical projection 20c to be interlocked with the hollow portion of each of the ports 6 and 7.
The port plugs 20 are attached to the ports 6 and 7 such that the ports 6 and 7 are covered to their bases as shown in Figure 5(B).
In this state, the port plugs can be subjected to sterilization described later to thereby maintain the sterilized states of the second space 12 and the lateral surfaces of the ports 6 and 7 until immediately before use of the hollow fiber membrane-based medical device.

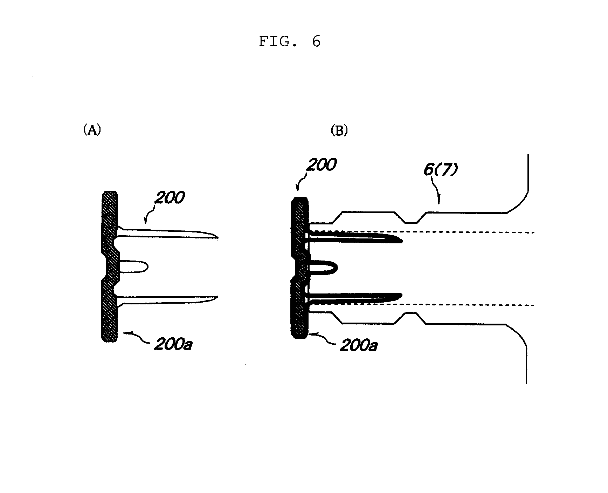

The port plugs 20 are not particularly limited by shape.
Specific examples thereof may include: as shown in Figure 5(A), wrapping-type plugs having a shape to wrap the whole port 6 or 7; and as shown as a plug 200 in Figures 6(A) and 6(B), squeezed-type plugs having a shape to produce hermeticity by contacting the partial surface of the port plug 200 with the inner wall surface in the internal space (second space 12) of the port 6 or 7.
As shown in Figure 5(A), the wrapping-type plugs having the shape to wrap the whole port 6 or 7 are superior in the function of maintaining the sterilized state. However, for the hollow fiber membrane-based medical device of the present embodiment, each plug attached to the ports 6 and 7 and the nozzles 4 and 5 of the headers 2 and 2' may be of any type, and plugs of the other types may be used.

In the hollow fiber membrane-based medical device of the present embodiment, the inlet and/or the outlet have a portion formed of a polymer material having a volume resistivity of 5 × 10¹³ Ω·cm or smaller, i.e., their respective predetermined plugs. The portion having the volume resistivity of 5 × 10¹³ Ω·cm or smaller may be a portion or the whole of each port plug 30.

Examples of materials for the port plugs 20, 200 include, but not limited to, polyethylene resins, polypropylene resins, polycarbonate resins, polystyrene resins, polyamide resins, polyacetal resins, and silicon resins. Particularly, the polyethylene resins and thepolypropylene resins are preferable because these resins have adhesion properties or plugging properties based on moderate hardness and are already versatile as wrapping-type plugs or squeezed-type plugs.
These resins may be used alone or in combination as long as the polymer material having the volume resistivity of 5 × 10¹³ Ω·cm or smaller can be obtained. Alternatively, these resins can be combined with an antistatic agent or a conductive polymer material to thereby more conveniently obtain materials for the plugs suitable for the purpose of the present invention.
Since the hollow fiber membrane-based medical device is basically used in contact with a body fluid, a method involving blending predetermined resins with antistatic agents and molding this into a predetermined component is more preferable than a method involving coating the surface of a constituent component with the antistatic agents, from a safety standpoint.
Examples of the antistatic agents to be blended with thermoplastic resin may include polymer-based antistatic agents such as antistatic agents having a polyoxyalkylene chain (e.g., Japanese Patent Laid-Open Nos. 03-255161 and 03-258850), antistatic agents comprising a copolymer of polyether having an oxyethylene group and polyether having no oxyethylene group, wherein the oxyethylene group moiety has a degree of crystallinity in a particular range (e.g., Japanese Patent Laid-Open No. 2009-108259), and antistatic agents (commercially available) comprising block polymer having polyether as a hydrophilic segment, surfactant-based antistatic agents, conductive fillers (carbon black, etc.), and conductive ABS resins.

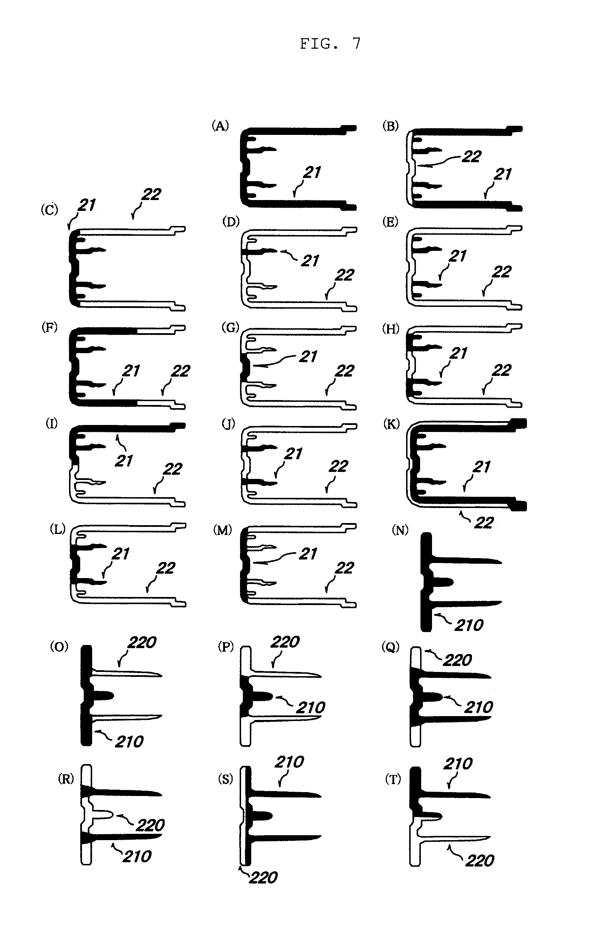

The portion formed of a polymer material having thye volume resistivity of 5 × 10¹³ Ω·cm or smaller, which constitutes each port plug 20 or 200, will be described with reference to specific examples shown in Figures 7(A) to 7(T).
In Figures 7(A) to 7(T), the portions represented by reference numerals 21 and 210 each depict a portion formed of a polymer material having the volume resistivity of 5 × 10¹³ Ω·cm or smaller, while the portions represented by reference numerals 22 and 220 depict a portion formed of a polymer material having the volume resistivity larger than 5 × 10¹³ Ω·cm.
Each of these portions may be composed of one component or a combination of a plurality of components.

In the hollow fiber membrane-based medical device of the present embodiment, electric charge accumulated in the main body due to electron beam irradiation during sterilization treatment described later is dissipated from the main body to thereby effectively prevent dielectric breakdown in the hollow fiber membrane-based medical device.
It is thus preferred that, in the constituent portion of each port plug, the portion having the volume resistivity of 5 × 10¹³ Ω·cm or smaller should be provided in contact with the second space 12 constituting the hollow fiber membrane-based medical device and also with the external space of the main body of the hollow fiber membrane-based medical device in a seamless way.
As a result, electric charge accumulated in the main body of the hollow fiber membrane-based medical device can be passed through only the portion having the volume resistivity of 5 × 10¹³ Ω·cm or smaller and dissipated to the external space of the main body of the hollow fiber membrane-based medical device.
Even if the portion having the volume resistivity of 5 ×10¹³ Ω·cm or smaller is not brought into contacted with the second space 12 in the hollow fiber membrane-based medical device or the external space of the main body, or both, this portion provided in a portion of the plug can sufficiently facilitate the dissipation, from the main body, of electric charge accumulated in the main body of the hollow fiber membrane-based medical device and contributes to the effective prevention of dielectric breakdown in the hollow fiber membrane-based medical device.

In the hollow fiber membrane-based medical device of the present embodiment, the constituent members other than the port plugs and the nozzle plugs may also be formed of a material having the volume resistivity of 5 × 10¹³ Ω·cm or smaller.
In this case, this material can be combined, if necessary, with the commercially available antistatic agent or conductive polymer material to thereby relatively easily achieve the volume resistivity of 5 × 10¹³ Ω·cm or smaller.

The volume resistivity, also called volume resistivity value, can be measured specifically at 22°C in a humidity environment of 60% RH using a test piece of 100 × 100 mm and a predetermined measurement apparatus, for example, ULTRA HIGH RESISTANCE METER R8340A, TEST FIXTURE R12702A manufactured by Advantest Corp. according to JIS K6911.
If the volume resistivity exceeds 5 × 10¹³ Ω·cm, electric charge accumulated in the main body of the hollow fiber membrane-based medical device due to electron beam irradiation performed for sterilization treatment cannot sufficiently be dissipated. The resulting accumulated electric charge easily causes dielectric breakdown in the hollow fiber membrane-based medical device.
The smaller the volume resistivity is, the more easily the electric charge accumulated in the main body of the hollow fiber membrane-based medical device can be dissipated, i.e., the more easily the effect of preventing dielectric breakdown can be obtained. It is thus preferred that at least a portion of each plug constituting the hollow fiber membrane-based medical device of the present embodiment should have a portion formed of a polymer material having small volume resistivity.
In this context, typical polymer materials having sufficiently small volume resistivity may present problems such as high production cost, small mechanical strength, and difficult attachment to each port. It is thus preferred to select a material according to the purpose.
For preventing dielectric breakdown without causing these problems, at least a portion of each plug constituting the inlet and/or the outlet in the hollow fiber membrane-based medical device has the volume resistivity of 5 × 10¹³ Ω·cm or smaller, preferably 1 × 10⁻² to 5 × 10¹³ Ω·cm, more preferably 5 × 10⁴ to 5 × 10¹³ Ω·cm.

The hollow fiber membrane-based medical device of the present embodiment is preferably constituted such that the port plug 20 or 200 has the portion formed of a polymer material having the volume resistivity of 5 × 10¹³ Ω·cm or smaller, at least in a partial top portion thereof (the top portions of the port plugs 20 and 200 are represented by 20a in Figures 5(A) and 5(B) and 200a in Figures 6(A) and 6(B), respectively).
In this context, the phrase "at least in a partial top portion thereof" may be a portion or the whole of the top portion of the plug and also means only the top portion or the top portion plus the other portion.

When the port plug 20 or 200 has the portion having the volume resistivity of 5 × 10¹³ Ω·cm or smaller, at least in a partial top portion thereof (in 20a or 200a), it is further preferred that this portion should be brought into contact on the outer surface side of the port plug 20 or 200 with the external space of the hollow fiber membrane-based medical device and on the inner surface side of the port plug with the second space 12 in a seamless way.
Examples of materials for the portion constituting at least the partial top portion (in 20a or 200a) and having the volume resistivity of 5 × 10¹³ Ω·cm or smaller may include, but not limited to, polyethylene resins, polypropylene resins, polycarbonate resins, polystyrene resins, polyamide resins, polyacetal resins, and silicon resins, as in the plugs. Particularly, polyethylene resins and polypropylene resins are preferable because these resins have adhesion properties or plugging properties based on moderate hardness and are already versatile as wrapping-type plugs or squeezed-type plugs.
These resins may be used alone or in combination as long as the polymer material having volume resistivity of 5 × 10¹³ Ω·cm or smaller can be obtained. Alternatively, these resins can be combined with an antistatic agent or a conductive polymer material to thereby more conveniently obtain materials suitable for the purpose of the present invention.
Examples of materials for the portion (member) other than the portion constituting at least the partial top portion (in 20a or 200a) and having the volume resistivity of 5 × 10¹³ Ω·cm or smaller may include polyethylene resins, polypropylene resins, polycarbonate resins, polystyrene resins, polyamide resins, polyacetal resins, and silicon resins. In terms of strength, it is preferred that this portion (member) should be formed of a polymer material having the volume resistivity larger than 5 × 10¹³ Ω·cm.
Particularly, polyethylene resins and polypropylene resins are preferable because these resins have adhesion properties or plugging properties based on moderate hardness and are already versatile as wrapping-type plugs or squeezed-type plugs.

In the examples shown in Figures 7(A) to 7(T), plugs constituted such that the port plug 20 or 200 has, at least in a partial top portion thereof, the portion having the volume resistivity of 5 × 10¹³ Ω·cm or smaller correspond to those shown in Figures 7(A), 7(C), 7(D), and 7(F) to 7(T) .

As described above, the low-volume resistivity portion constituting each port plug and formed of a polymer material having vthe olume resistivity of 5 × 10¹³ Ω·cm or smaller is provided in contact with the second space 12 constituting the hollow fiber membrane-based medical device and also with the external space of the main body of the hollow fiber membrane-based medical device in a seamless way. This constitution can produce the effect of more efficiently and reliably preventing dielectric breakdown.
Specifically, electric charge accumulated in the main body of the hollow fiber membrane-based medical device can be passed through only the low-volume resistivity portion formed of a polymer material having volume resistivity of 5 × 10¹³ Ω·cm or smaller and dissipated to the external space of the main body of the hollow fiber membrane-based medical device.
As a result, dielectric breakdown can be prevented. In addition, change only in the volume resistivity of the port plug portion suffices without the need for reducing the whole volume resistivity of the components of the hollow fiber membrane-based medical device. The resulting hollow fiber membrane-based medical device can maintain practically sufficient strength as a whole, can prevent increase in eluted materials, and can overcome the problems associated with dielectric breakdown, which have been tackled by the conventional techniques.

For the hollow fiber membrane-based medical device of the present embodiment, it is preferred that the low-volume resistivity portion constituting each port plug and formed of a polymer material having the volume resistivity of 5 × 10¹³ Ω·cm or smaller should be limited only to the top (represented by 20a in Figures 5(A) and 5(B) or 200a in Figures 6(A) and (B)) portion of the port plug.
For effectively preventing the occurrence of dielectric breakdown attributed to electron beam irradiation, it is preferred to increase the size of the low-volume resistivity portion formed of a polymer material having the volume resistivity of 5 × 10¹³ Ω·cm or smaller. However, typical polymer materials having the volume resistivity of 5 × 10¹³ Ω·cm or smaller may present problems such as high production cost, small mechanical strength derived from increase in occupied volume in the port plug, and difficult attachment to each port.
Moderate hardness leading to plugging properties or moderate adhesion properties can be obtained by controlling as described above the low-volume resistivity portion formed of a polymer material having volume resistivity of 5 × 10¹³ Ω·cm or smaller.
From the viewpoint of also obtaining sufficient strength, it is preferred that only the whole or a portion of the top portion 20a or 200a should have such low volume resistivity and that the portion other than the top portion of the port plug should be formed of a polymer material having the volume resistivity larger than 5 × 10¹³ Ω·cm.

The port plug 20 or 200 can be prepared by a molding method conventionally known in the art, for example, an injection molding method.
When the port plug 20 or 200, as described above, has the low-volume resistivity portion having the volume resistivity of 5 × 10¹³ Ω·cm or smaller, at least in a partial top portion thereof (in 20a or 200a), the port plug can be prepared, for example, by a two-color molding method so that the port plug has in combination portions differing in volume resistivity. Alternatively, the low-volume resistivity portion constituting at least the partial top portion (in 20a or 200a) and the other portion may be injection-molded separately to prepare their respective components, which are combined in downstream operation to prepare the port plug.

### (Type of hollow fiber membrane-based medical device)

In general, hollow fiber membrane-based medical devices are broadly classified into two types: wet type in which the hollow inside of the hollow fiber membrane or the gap between the hollow fiber membrane and a container is filled with an aqueous medium; and non-wet type in which such space is not filled with an aqueous medium.
The hollow fiber membrane-based medical devices of the latter type may be categorized as dry type having a hollow fiber membrane with a low water content, i.e., a small percent of water, and semidry type (also called half-wet type) having a membrane wetted to some extent with water, a humectant, or the like.
The hollow fiber membrane-based medical device of the present embodiment can be prepared as any type of hollow fiber membrane-based medical device according to the purpose.

When the hollow fiber membrane-based medical device of the present embodiment is of wet type, an antioxidant or buffer mixed with pure water or a solvent can be generally used as an aqueous medium.
Examples of the antioxidant may include, but not particularly limited to, sodium pyrosulfite, acetone sodium bisulfite, sodium formaldehyde sulfoxylate, sodium hydrosulfite, and 1-ascorbic acid. Examples of the buffer may include, but not particularly limited to, phosphate buffers, trishydroxymethylaminomethane buffers, acetate buffers, citrate buffers, and borate buffers.
Examples of the solvent may include, but not particularly limited to, methanol, ethanol, glycerin, propylene glycol, and isopropanol.
When the hollow fiber membrane-based medical device of the present embodiment is of semidry type, a humectant can be used preferably, which has the function of protecting a hydrophilic polymer from deterioration, and which is easily carried on a membrane surface by virtue of moderate viscosity, and simultaneously satisfies the requirements that: it does not form a strong chemical bond with a hydrophobic polymer or a hydrophilic polymer; and it is easily washed off with a physiological aqueous solution.
Examples thereof may include polyhydric alcohols such as glycerin, mannitol, glycols (e.g., ethylene glycol, diethylene glycol, propylene glycol, and tetraethylene glycol), and polyglycols (e.g., polyethylene glycol).
Particularly, an aqueous solution of glycerin or polyethylene glycol is more preferable in terms of its actual achievements as pore size-retaining agents or surface modifiers for hollow fiber membranes for blood purification. An aqueous glycerin solution is particularly preferable, though the humectant according to the present invention is not particularly limited to them.

### (Sterilization treatment)

The hollow fiber membrane-based medical device of the present embodiment is sterilized by electron beam irradiation.
The irradiation dose of the electron beams is preferably 5 to 60 kGy (kilogray), more preferably 15 to 50 kGy, further preferably 15 to 45 kGy.
The feature of the hollow fiber membrane-based medical device of the present embodiment is that the sterilization treatment is performed in a state where the port plugs 20 and the nozzle plugs 30 are attached to the ports 6 and 7 on the side of the tubular container 9 and the nozzles 4 and 5 of the headers 2 and 2', respectively.
It is also preferred to perform the sterilization treatment by electron beam irradiation in a state where the hollow fiber membrane-based medical device is packaged in a sterilization bag.
The sterilization treatment may be performed in a state where the hollow fiber membrane-based medical device is not packaged in a sterilization bag or in a state where the hollow fiber membrane-based medical device without the plugs attached thereto is packaged in a sterilization bag. From the viewpoint of maintaining the sterilized state of the hollow fiber membrane-based medical device until immediately before its use or for immediately using the hollow fiber membrane-based medical device in medical practice, it is preferred to perform the sterilization treatment in a state where the hollow fiber membrane-based medical device with the plugs attached thereto is packaged in a sterilization bag.

Examples of materials for the sterilization bag for use as a package in the sterilization treatment may include polyvinylidene chloride films, polyvinyl alcohol films, biaxial stretched polyvinyl alcohol films, polyvinylidene chloride-coated biaxial stretched polyalcohol films, polyester/aluminum/polyethylene laminate sheets, aluminum foils, and films having a polyethylene/nylon bilayer structure.
Particularly, a method for sealing a film having a polyethylene/nylon bilayer structure by lamination is preferable from the viewpoint of bag productivity or cost, though the sterilization bag according to the present invention is not particularly limited to them.

### [Field of utilization of medical device]

The medical device of the present embodiment can be used in a wide range of applications.
Examples thereof may include hemodialyzers, hemodialysis filters, hemofilters, continuous hemodialysis filters, continuous hemofilters, plasma separators, plasma fractionators, plasma component adsorbers, virus removers, virus adsorbers, blood concentrators, plasma concentrators, ascitic fluid filters, and ascitic fluid concentrators. The medical device of the present embodiment can be applied to various medical devices utilizing filtration characteristics or selective adsorption characteristics (involving hollow fiber membranes on which ligands that exhibit interaction with substances to be adsorbed are immobilized) possessed by the hollow fiber membrane.

### Examples

Hereinafter, the present invention will be described with reference to specific Examples and Comparative Examples thereto. However, the present invention is not intended to be limited to Examples below.
First, various measurement methods used in Examples will be described.

### [Measurement of incidence of dielectric breakdown]

### (Visual observation)

The presence or absence of a black burnt deposit was visually confirmed as to a spot at which dielectric breakdown occurred in each hollow fiber membrane-based medical device due to electron beam irradiation.

### (In-water positive pressure test)

Each hollow fiber membrane-based medical device was completely immersed in water. A pressure of 150 kPa was applied to the hollow fiber membrane-based medical device by air supply to the inside thereof, while the hollow fiber membrane-based medical device was kept for 30 seconds. The occurrence of air bubbles from a dielectric breakdown spot was observed to confirm the presence or absence of dielectric breakdown.
This in-water positive pressure test was conducted for each of the first and second spaces in the hollow fiber membrane-based medical device.
When the occurrence of dielectric breakdown was confirmed by either of the methods described in (Visual observation) and (In-water positive pressure test) above, the hollow fiber membrane-based medical device was determined to have dielectric breakdown. The ratio of the number of hollow fiber membrane-based medical devices having dielectric breakdown to the total number of prepared hollow fiber membrane-based medical devices was indicated by percentage.

### [Method for measuring volume resistivity of plug]

Materials constituting port plugs of Examples 1 to 3 and Comparative Examples 1 to 2 described later and portions having predetermined low volume resistivity or materials constituting port plugs of Examples 4 to 7 and Comparative Examples 3 to 5 were separately processed into a test piece of 100 × 100 mm. The test piece was assayed at 22°C in a humidity environment of 60% RH using "ULTRA HIGH RESISTANCE METER R8340A, TESTFIXTURE R12702A" manufactured by Advantest Corp. according to JIS K6911.

### [Example 1]

Polysulfone (manufactured by Solvay Advanced Polymers, P-1700): 17 parts by mass
PVP (manufactured by ISP Ltd., K-90): 4 parts by mass
Dimethylacetamide (hereinafter, referred to as DMAC): 79 parts by mass
These components were used to prepare a uniform dope.
42% aqueous solution of DMAC was used as an inner solution and discharged from a spinneret together with the dope.
The amounts of the dope and the inner solution discharged were adjusted such that the membrane thickness and the inner diameter were 35 µm and 185 µm, respectively, after drying.
The discharged dope was dipped in a coagulation bath (containing water) of 60°C positioned 50 cm below the spinneret. After a coagulation step at a rate of 30 m/min and a washing step using water, the hollow fiber membrane was introduced to a dryer and dried at 160°C. Then, the crimped polysulfone-containing hollow fiber membrane was wound up.
Next, a bundle of the wound-up hollow fiber membrane of 16000 filaments was loaded in a tubular container (made of a styrene-butadiene copolymer (manufactured by Asahi Kasei Chemicals Corp., registered trademark: Asaflex)) designed such that the hollow fiber membrane had an effective membrane area of 2.5 m². Both ends of the bundle were fixed to both ends of the tubular container, respectively, by bonding with a urethane resin (manufactured by Sanyo Kasei Industries, Ltd.). The surfaces at both ends were cut so that the hollow portion of the hollow fiber membrane was open at both ends.
From the open end, an aqueous solution of glycerin (manufactured by Wako Pure Chemical Industries, Ltd., special grade) with a concentration of 63% was injected into the hollow fiber membrane for 5 seconds. After flash with 0.2 MPa air for 10 seconds, both ends were capped with headers made of a styrene-butadiene copolymer (manufactured by Asahi Kasei Chemicals Corp., registered trademark: Asaflex).
Nozzles for blood influx/efflux were capped with nozzle plugs made of polyethylene. A port on the main body was capped with a port plug formed of one component molded from a polyethylene (manufactured by Prime Polymer Co., Ltd., registered trademark: HI-ZEX) chip and a polyethylene-b-polyether block polymer (manufactured by Sanyo Kasei Industries, Ltd., Pelestat) chip added at a mixing ratio of 87%:13% to an injection molding machine.
Then, the resulting device was packaged in a sterilization bag having a polyethylene/nylon bilayer structure and irradiated with an electron beam at an irradiation dose of 35 kGy to prepare a semidry-type hollow fiber membrane-based medical device.
Table 1 below shows the incidence of dielectric breakdown in this hollow fiber membrane-based medical device and the volume resistivity of the port plug attached to the port on the main body.

### [Example 2]

A semidry-type hollow fiber membrane-based medical device was obtained under the same conditions as in Example 1 except that a port on the main body was capped with a port plug formed of one component molded from polyolefin resin mixed with conductive carbon.
Table 1 below shows the incidence of dielectric breakdown in this hollow fiber membrane-based medical device and the volume resistivity of the port plug attached to the port on the main body.

### [Example 3]

A semidry-type hollow fiber membrane-based medical device was obtained under the same conditions as in Example 1 except that:
a port on the main body was capped with a port plug formed of one component molded from a polyethylene (manufactured by Prime Polymer Co., Ltd., registered trademark: HI-ZEX) chip and a polyethylene-b-polyether block polymer (manufactured by Sanyo Kasei Industries, Ltd., Pelestat) chip added at a mixing ratio of 85%:15% to an injection molding machine; and the device was irradiated with an electron beam at an irradiation dose of 57 kGy.
Table 1 below shows the incidence of dielectric breakdown in this hollow fiber membrane-based medical device and the volume resistivity of the port plug attached to the port on the main body.

### [Comparative Example 1]

A semidry-type hollow fiber membrane-based medical device was obtained under the same conditions as in Example 1 except that
a port on the main body was capped with a port plug formed of one component made of polyethylene (manufactured by Prime Polymer Co., Ltd., registered trademark: HI-ZEX).
Table 1 below shows the incidence of dielectric breakdown in this hollow fiber membrane-based medical device and the volume resistivity of the port plug attached to the port on the main body.

### [Comparative Example 2]

A semidry-type hollow fiber membrane-based medical device was obtained under the same conditions as in Example 1 except that
a port on the main body was capped with a port plug formed of one component molded from a polyethylene (manufactured by Prime Polymer Co., Ltd., registered trademark: HI-ZEX) chip and a polyethylene-b-polyether block polymer (manufactured by Sanyo Kasei Industries, Ltd., Pelestat) chip added at a mixing ratio of 92%:8% to an injection molding machine.
Table 1 below shows the incidence of dielectric breakdown in this hollow fiber membrane-based medical device and the volume resistivity of the port plug attached to the port on the main body.

**[Table 1]**

| | Irradiation dose | Incidence of dielectric breakdown | Volume resistivity (Ω·cm) |
|---|---|---|---|
| Example 1 | 35kGy | 0% | 3.4 × 10¹³ |
| Example 2 | 35kGy | 0% | 8.2 × 10⁴ |
| Example 3 | 57kGy | 0% | 9.0 × 10¹² |
| Comparative Example 1 | 35kGy | 73% | 4.5 × 10¹⁷ |
| Comparative Example 2 | 35kGy | 40% | 4.0 × 10¹⁴ |

As shown in Table 1, the hollow fiber membrane-based medical devices of Examples 1 to 3 having the plug formed of a polymer material having volume resistivity of 5 × 10¹³ Ω·cm or smaller were all able to effectively prevent the occurrence of dielectric breakdown.

### [Example 4]

Polysulfone (manufactured by Solvay Advanced Polymers, P-1700): 17 parts by mass
PVP: polyvinylpyrrolidone (manufactured by ISP Ltd., K-90): 4 parts by mass
Dimethylacetamide (hereinafter, referred to as DMAC): 79 parts by mass
These components were used to prepare a uniform dope.
An aqueous solution containing 42% by mass of DMAC was used as an inner solution and discharged from a spinneret together with the dope.
The amounts of the dope and the inner solution discharged were adjusted such that the membrane thickness and the inner diameter were 35 µm and 185 µm, respectively, after drying.
The discharged dope was dipped in a coagulation bath (containing water) of 60°C positioned 50 cm below the spinneret. After a coagulation step at a rate of 30 m/min and a washing step using water, the hollow fiber membrane was introduced to a dryer and dried at 160°C. Then, the crimped polysulfone-containing hollow fiber membrane was wound up.
Next, a bundle of the wound-up hollow fiber membrane of 16000 filaments was loaded in a tubular container (made of a styrene-butadiene copolymer (manufactured by Asahi Kasei Chemicals Corp., registered trademark: Asaflex)) designed such that the hollow fiber membrane had an effective membrane area of 2.5 m². Both ends of the bundle were fixed to both ends of the tubular container bonding with a urethane resin (manufactured by Sanyo Kasei Industries, Ltd.), respectively. The surfaces at both ends were cut so that the hollow portion of the hollow fiber membrane was open at both ends.
From the open end, an aqueous solution of glycerin (manufactured by Wako Pure Chemical Industries, Ltd., special grade) with a concentration of 63% was injected into the hollow fiber membrane for 5 seconds. After flash with 0.2 MPa air for 10 seconds, both ends were capped with headers made of a styrene-butadiene copolymer (manufactured by Asahi Kasei Chemicals Corp., registered trademark: Asaflex).
Nozzles for influx/efflux of a solution to be treated were capped with nozzle plugs made of polyethylene. Ports 6 and 7 on the main body were capped with port plugs having a shape shown in [Figure 7(G)]. The portion represented by reference numeral 21, which was a partial top portion of the port plug in [Figure 7(G)], was formed of one component molded from a polyethylene (manufactured by Prime Polymer Co., Ltd., registered trademark: HI-ZEX) chip and a polyethylene-b-polyether block polymer (manufactured by Sanyo Kasei Industries, Ltd., Pelestat) chip added at a mixing ratio of 87%:13% by mass to an injection molding machine. The portion represented by reference numeral 22 was formed of one component made of polyethylene (manufactured by Prime Polymer Co., Ltd., registered trademark: HI-ZEX).
Then, the resulting device was packaged in a sterilization bag having a polyethylene/nylon bilayer structure and irradiated with an electron beam at an irradiation dose of 35 kGy to prepare a semidry-type hollow fiber membrane-based medical device.
Table 2 below shows the incidence of dielectric breakdown in this hollow fiber membrane-based medical device and the volume resistivity of the portion represented by reference numeral 21 (low-volume resistivity portion; the same holds true for the description below) in the port plugs attached to the ports on the main body.

### [Example 5]

A semidry-type hollow fiber membrane-based medical device was obtained under the same conditions as in Example 4 except that
ports 6 and 7 on the main body were capped with port plugs having a shape shown in [Figure 7(G)], wherein the portion represented by reference numeral 21, which was a partial top portion of the port plug in [Figure 7(G)], was formed of one component molded from polyolefin resin mixed with conductive carbon, and the portion represented by reference numeral 22 was formed of one component made of polyethylene (manufactured by Prime Polymer Co., Ltd., registered trademark: HI-ZEX).
Table 2 below shows the incidence of dielectric breakdown in this hollow fiber membrane-based medical device and the volume resistivity of the portion represented by reference numeral 21 in the port plugs attached to the ports on the main body.

### [Example 6]

A semidry-type hollow fiber membrane-based medical device was obtained under the same conditions as in Example 4 except that
the irradiation dose of electron beams was changed to 57 kGy.
Table 2 below shows the incidence of dielectric breakdown in this hollow fiber membrane-based medical device and the volume resistivity of the portion represented by reference numeral 21 in the port plugs attached to the ports on the main body.

### [Example 7]

A semidry-type hollow fiber membrane-based medical device was obtained under the same conditions as in Example 4 except that:
ports 6 and 7 on the main body were capped with port plugs having a shape shown in [Figure 7(C)], wherein
the portion represented by reference numeral 21, which was the top portion of the port plug in [Figure 7(C)], was formed of one component molded from polyolefin resin mixed with conductive carbon, and the portion represented by reference numeral 22 was formed of one component made of polyethylene (manufactured by Prime Polymer Co., Ltd., registered trademark: HI-ZEX); and
then, the resulting device was packaged in a sterilization bag having a polyethylene/nylon bilayer structure and irradiated with an electron beam at an irradiation dose of 57 kGy.
Table 2 below shows the incidence of dielectric breakdown in this hollow fiber membrane-based medical device and the volume resistivity of the portion represented by reference numeral 21 in the port plugs attached to the ports on the main body.

### [Comparative Example 3]

A semidry-type hollow fiber membrane-based medical device was obtained under the same conditions as in Example 4 except that
ports 6 and 7 on the main body were capped with port plugs having a shape shown in [Figure 7(G)], wherein
the portion represented by reference numeral 21, which was a partial top portion of the port plug in [Figure 7(G)], was formed of one component molded from a polyethylene (manufactured by Prime Polymer Co., Ltd., registered trademark: HI-ZEX) chip and a polyethylene-b-polyether block polymer (manufactured by Sanyo Kasei Industries, Ltd., Pelestat) chip added at a mixing ratio of 92%:8% by mass to an injection molding machine, and the portion represented by reference numeral 22 was formed of one component made of polyethylene (manufactured by Prime Polymer Co., Ltd., registered trademark: HI-ZEX).
Table 2 below shows the incidence of dielectric breakdown in this hollow fiber membrane-based medical device and the volume resistivity of the portion represented by reference numeral 21 in the port plugs attached to the ports on the main body.

### [Comparative Example 4]

A semidry-type hollow fiber membrane-based medical device was obtained under the same conditions as in Example 4 except that
ports 6 and 7 on the main body were capped with port plugs having a shape shown in [Figure 7(C)], wherein
the portion represented by reference numeral 21, which was the top portion of the port plug in [Figure 7(C)], was formed of one component molded from a polyethylene (manufactured by Prime Polymer Co., Ltd., registered trademark: HI-ZEX) chip and a polyethylene-b-polyether block polymer (manufactured by Sanyo Kasei Industries, Ltd., Pelestat) chip added at a mixing ratio of 92%:8% by mass to an injection molding machine, and the portion represented by reference numeral 22 was formed of one component made of polyethylene (manufactured by Prime Polymer Co., Ltd., registered trademark: HI-ZEX).
Table 2 below shows the incidence of dielectric breakdown in this hollow fiber membrane-based medical device and the volume resistivity of the portion represented by reference numeral 21 in the port plugs attached to the ports on the main body.

### [Comparative Example 5]

A semidry-type hollow fiber membrane-based medical device was obtained under the same conditions as in Example 4 except that
ports 6 and 7 on the main body were capped with port plugs each formed of one component made of polyethylene (manufactured by Prime Polymer Co., Ltd., registered trademark: HI-ZEX), wherein
the shape of each port plug was the same as in [Figure 7(C) or 7(G)], while the whole port plug was made of polyethylene only.
Table 2 below shows the incidence of dielectric breakdown in this hollow fiber membrane-based medical device and the volume resistivity of the port plugs attached to the ports on the main body.

**[Table 2]**

| | Shape of plug | Irradiation dose | Incidence of dielectric breakdown | Volume resistivity (Ω·cm) |
|---|---|---|---|---|
| Example 4 | [Figure 7] (G) | 35kGy | 0% | 3.4 × 10¹³ |
| Example 5 | [Figure 7] (G) | 35kGy | 0% | 8.2 × 10⁴ |
| Example 6 | [Figure 7] (G) | 57kGy | 0% | 3.4 × 10¹³ |
| Example 7 | [Figure 7] (C) | 57kGy | 0% | 8.2 × 10⁴ |
| Comparative Example 3 | [Figure 7] (G) | 35kGy | 39% | 4.0 × 10¹⁴ |
| Comparative Example 4 | [Figure 7] (C) | 35kGy | 17% | 4.0 × 10¹⁴ |
| Comparative Example 5 | - | 35kGy | 73% | 4.5 × 10¹⁷ |

As shown in Table 2 above, the medical devices of Examples 4 to 7 were all able to effectively prevent the occurrence of dielectric breakdown.

### Industrial Applicability

A medical device of the present invention has industrial applicability as various medical devices for hemodialysis, hemodiafiltration, hemofiltration, continuous hemodiafiltration, continuous hemofiltration, plasma exchange, double filtration plasma exchange, virus removal, blood concentration, ascitic fluid filtration/concentration, adsorption, etc., in medical practice.

### Reference Signs List

1 Hollow fiber membrane-based medical device
2 and 2' Header
3 Hollow fiber membrane
4 and 5 Nozzle
6 and 7 Port
8 Potting resin
9 Tubular container
11 First space
12 Second space
20 and 200 Port plug to be attached to port
20a Top portion of port plug
20b Cylindrical projection to be interlocked with lateral surface of each port
20c Cylindrical projection to be interlocked with hollow portion of each port
200a Top portion of port plug
21 and 210 In constituent portion of plug to be attached to port, a portion formed of polymer material having volume resistivity of 5 × 10¹³ Ω·cm or smaller
22 and 220 In constituent portion of plug to be attached to port, a portion formed of polymer material having volume resistivity larger than 5 × 10¹³ Ω·cm
30 Nozzle plug to be attached to nozzle

## Claims

1. A hollow fiber membrane-based medical device comprising:
a tubular container;
a bundle of a hollow fiber membrane loaded along a longitudinal direction of the tubular container and having both ends fixed to both ends of the tubular container, respectively;
a potting resin embedding both ends of the bundle of the hollow fiber membrane, respectively, so that the ends are fixed to the both ends of the tubular container, respectively;
a header facing both end faces of the hollow fiber membrane and provided at the both ends of the tubular container, respectively, the header having a nozzle serving as a fluid inlet and/or outlet;
a port provided on a side of the tubular container and serving as a fluid inlet and/or outlet;
a nozzle plug removably attached to the respective nozzles; and
a port plug removably attached to the port,
wherein the port plug has a portion formed of a polymer material having a volume resistivity of 5 × 10¹³ Ω·cm or smaller, and
the hollow fiber membrane-based medical device is irradiated with an electron beam.

2. The hollow fiber membrane-based medical device according to claim 1, wherein the port plug is formed of a polymer material having a volume resistivity of 5 × 10¹³ Ω·cm or smaller.

3. The hollow fiber membrane-based medical device according to claim 1 or 2, wherein the nozzle plug have a portion formed of a polymer material having a volume resistivity of 5 × 10¹³ Ω·cm or smaller.

4. The hollow fiber membrane-based medical device according to any one of claims 1 to 3, wherein the nozzle plug is formed of a polymer material having a volume resistivity of 5 × 10¹³ Ω·cm or smaller.

5. The hollow fiber membrane-based medical device according to any one of claims 1 to 4, wherein the port plug has, at least in a partial top portion thereof, the portion formed of a polymer material having a volume resistivity of 5 × 10¹³ Ω·cm or smaller.

6. The hollow fiber membrane-based medical device according to any one of claims 1 to 5, further comprising
a first space between an internal side of the hollow fiber membrane and an inner surface of the header, and
a second space between an external side of the hollow fiber membrane and an inner surface of the tubular container,
wherein the port plug has, at least in a partial top portion thereof, the portion formed of a polymer material having a volume resistivity of 5 × 10¹³ Ω·cm or smaller, wherein the portion is brought into contact on an outer surface side of the port plug with an external space of the hollow fiber membrane-based medical device and on an inner surface side of the port plug with the second space in a seamless way.

7. The hollow fiber membrane-based medical device according to any one of claims 1 to 6, wherein the port plug has, only in a top portion thereof, the portion formed of a polymer material having a volume resistivity of 5 × 10¹³ Ω·cm or smaller.

8. A medical device comprising:
a container having an inner space molded with an insulating material;
a filter medium loaded in the inner space;
an inlet provided on a container wall surface which defines the inner space and flowing a fluid into the container;
an outlet provided on a container wall surface which defines the inner space and effusing a fluid from the container,
wherein the inlet and/or the outlet have a portion formed of a polymer material having a volume resistivity of 5 × 10¹³ Ω·cm or smaller, and
the medical device is irradiated with an electron beam.
